# EUROPEAN PATENT SPECIFICATION

(11) **EP 0 696 263 B1**
(45) Date of publication and mention of the grant of the patent: **27.08.1997**
(21) Application number: 94915148.4
(22) Date of filing: 26.04.1994
(51) Int. Cl.: C07C 2/34, B01J 31/22

(54) **CATALYST COMPOSITION FOR ALKENE OLIGOMERISATION AND CO-OLIGOMERISATION**
KATALYTISCHE ZUSAMMENSETZUNG FÜR DIE OLIGOMERISIERUNG UND DIE CO-OLIGOMERISIERUNG VON ALKENEN
COMPOSITION DE CATALYSEUR POUR L'OLIGOMERISATION ET LA CO-OLIGOMERISATION D'ALCENES

(30) Priority: 28.04.1993 EP 93201229
(43) Date of publication of application: 14.02.1996
(73) Proprietor: SHELL INTERNATIONALE RESEARCH MAATSCHAPPIJ B.V., 2596 HR Den Haag (NL)
(72) Inventor: DOYLE, Michael, John, NL-1031 CM Amsterdam (NL); HEERES, Hero, Jan, NL-1031 CM Amsterdam (NL)
(86) International application number: EP9401430
(87) International publication number: WO9425416

(56) References cited:
- EP-A- 0 443 686
- EP-A- 0 447 880
- WO-A-91/09882

## Description

This invention relates to a novel catalyst composition and to its use in the oligomerisation and co-oligomerisation of one or more alkenes, in particular alpha alkenes. More in particular, the invention relates to the oligomerisation of ethene.

Polymerisation processes of olefins, including alkenes, such as the production of polyethylene from ethene, whereby soluble catalyst systems of the Ziegler-Natta type are used, are well known. In particular, oligomerisation processes of lower olefins to higher olefins are also well known. For example, from GB-A-1353873 it is known that C₄-C₂₀ linear alpha olefins can be prepared from ethene by oligomerisation in the presence of a nickel containing catalyst. The product linear alpha olefins, in particular those having 6-10 carbon atoms, are in great demand as intermediates in the preparation of detergents, lubricant additives and polyolefins.

However, the oligomerisation reaction also produces less valuable products, such as internal olefins and branched olefins and olefins having a number of carbon atoms outside the range of 4-24. By further processing, these by-products can be converted to the desired linear alpha olefins.

EP-A-0277003 and EP-A-0277004 both disclose catalyst compositions for the polymerisation and copolymerisation of olefins, comprising a combination of a first component which is a bis(cyclopentadienyl)titanium, zirconium or hafnium compound containing a substituent which is attached to the metal and which is capable of reacting with a cation, and a second component which is a compound having a bulky and labile anion which is substantially non-coordinating under the reaction conditions and a cation, capable of donating a proton. The two cyclopentadienyl groups may be substituted. The products according to these documents are true polymers, having a molecular weight of above 100000 (when determined in the Examples). Consequently, no attention is paid to properties which are important to olefins having a number of carbon atoms of less than 30, such as linearity and the position of the double bond.

The present Applicant's EP-A-0443686 and EP-B-0526943 both disclose methods for the co-oligomerisation of ethene with at least one other alpha olefin in the presence of a catalyst composition as broadly described above, wherein one or both of the cyclopentadienyl groups may be substituted. The preferred and only exemplified substituted ligand pair is bis-pentmethylcyclopentadienyl. The product oligomers still contain a substantial proportion of the less valuable branched olefins and internal olefins.

It has now been found that catalyst compositions as described above, wherein the ligand pair is bis-tetramethylcyclopentadienyl, are particularly useful in the oligomerisation or co-oligomerisation of ethene and other lower alkenes to higher alkenes, in that they effect a more preferential production of the linear alpha alkenes of the range having 4-24 carbon atoms and more in particular 6-10 carbon atoms.

Accordingly, the present invention provides a catalyst composition comprising
- a first component which is a substituted bis(cyclopentadienyl)titanium, zirconium or hafnium compound, also containing a substituent which is attached to the metal and which is capable of reacting with a cation and
- a second component which is a ionic combination of a bulky anion containing a plurality of boron atoms which is substantially non-coordinating under the reaction conditions employed with a cation,
characterized in that the substituted bis-cyclopentadienyl ligand pair is bis-tetramethylcyclopentadienyl.

The first component of the catalyst composition according to the invention is preferably a compound of the general formula

(Cp*)₂MR₁R₂,

wherein (Cp*)₂ is the bis-tetramethylcyclopentadienyl ligand pair, M is a metal chosen from the group of titanium, zirconium or hafnium and R₁ and R₂ are identical or different and selected from unsubstituted or substituted hydrocarbyl groups, hydrogen and halogen.

Preferably, R₁ and R₂ are alkyl groups, typically of from 1 to 5 carbon atoms, such as methyl.

Preferred metals are zirconium or hafnium.

The most preferred first component of the catalyst composition according to the present invention is bis-tetramethylcyclopentadienyl zirconium dimethyl.

Complexes such as the first component according to the invention can be prepared for example by the routes described in "Chemistry of Organo-Zirconium and Hafnium Compounds", by Lappert et al., 1986, published by John Wiley & Sons, Chichester, England.

The second component of the catalyst composition according to the invention is an ionic combination of a bulky anion containing a plurality of boron atoms and a proton-donating cation, the anion being such that it is substantially non-coordinating under the reaction conditions employed. Thus, it is intended that the anion should not coordinate, or at least coordinate only weakly, to the bis(cyclopentadienyl) metal entity of the first component. The boron-containing non-coordinating anion is preferably a carborate anion, suitably a carborate anion of the formula B₁₁CH₁₂-. Such carborates are known and can be prepared by methods such as that of Shelly et al, J. Am. Chem. Soc. 107 (1985) 5955-5959. Other bulky boron containing anions may also be used, such as a tetrakisrfluorophenyl) boron anion. The proton-donating cation is preferably a quaternary ammonium cation such as a trialkylammonium cation, for example tri-n-butylammonium cation.

Alternatively a cation may be used which is not proton-donating, such as a metal cation e.g. a silver ion, or a triphenylcarbenium ion.

The catalyst composition may be formed by mixing together the two components, preferably in solution in a suitable solvent such as toluene, chlorobenzene, an alkane or an alkene, to form a liquid catalyst system. The two components are generally employed in substantially equimolar amounts, although the molar ratio of the first component to the second component may vary within the range of from 0.1 to 5.0. Such a quantity of the catalyst system is usually employed in the reaction mixture as to contain from 10⁻¹ to 10⁻⁷ gram atoms, in particular from 10⁻³ to 10⁻⁵ gram atoms, of the metal per mole of olefin to be reacted.

The catalyst composition may be formed prior to its introduction to the reaction vessel, or it may be formed in situ.

The invention also provides the use of this catalyst composition in oligomerisation or co-oligomerisation, in particular for the preparation of linear alpha alkenes having 4-24 carbon atoms by oligomerisation or co-oligomerisation of ethene and/or an alpha alkene having 3-10 carbon atoms. The oligomerisation reaction according to the invention can be carried out in batch or continuous operation.

The oligomerisation reaction is generally, although not necessarily, carried out in an inert liquid which is suitably also the solvent for the catalyst components. The reaction is suitably carried out at an elevated temperature, preferably in the range of from 20 to 175 °C, more preferably at 50 to 150 °C. The reaction is suitably carried out under conditions of moderately elevated pressure, preferably in the range of from 100 to 10000 kPa, more preferably from 500 to 6000 kPa. The optimum conditions of temperature and pressure used in a particular reaction system in order to maximise the yield of the desired linear alpha alkenes can be readily established by those skilled in the art, but it has been found that conditions of between 70-120 °C and between 1000-3000 kPa are particularly advantageous in this respect with the catalyst systems of the present invention.

The starting reactants may be supplied to the reactor together with an inert diluent, such as nitrogen or helium when the reactant is gaseous, and a liquid solvent, e.g. the same solvent as that of the catalyst components, when the reactant is in the liquid form.

The reaction is preferably carried out in the absence of air or moisture.

Reaction times of from 1 minute to 5 hours have been found to be suitable, depending on the activity of the catalyst system and on the reaction conditions. After a suitable reaction time, a conventional catalyst deactivating agent such as water, methanol, or another alcohol, may be added if desired to the reaction mixture in order to terminate the reaction. Alternatively, the reaction can simply be terminated by the introduction of air.

The product mixed alkenes are preferentially linear alpha alkenes having a chain length within the range of 5 to 24 carbon atoms, of which those having between 6 and 10 carbon atoms in the chain are particularly preferred. They may be suitably recovered by distillation and separation techniques known in the art.

If desired, unconverted starting material and oligomeric products having a molecular weight outside the desired molecular weight may be recovered, processed if necessary and recycled to be used as starting material in a subsequent oligomerisation reaction.

The invention will be further illustrated by the following examples.

### Examples 1 to 4: oligomerisation of ethene

The procedures were carried out with rigorous exclusion of oxygen and moisture.

Catalyst liqours were prepared by dissolving in toluene, as First Component, in 30 ml, 0.25 mmol of (Cp*)₂MR₂, wherein M is zirconium, R₂ is dimethyl and (Cp*)₂ is:
in comparative Example 1: bis-pentamethylcyclopentadienyl;
in comparative Example 2: bis-methylcyclopentadienyl;
in comparative Example 3: bis-cyclopentadienyl;
in Example 4: bis-tetramethylclopentadienyl;
and as Second Component, in 70 ml, in all cases 0.05 mmol of Bu₃NHB₁₁CH₁₂ (tri-n-butylammonium 1-carbadodecaborate).

The Second Component of the catalytic composition was first added to an autoclave of 500 ml, followed by pressurising the autoclave with ethene to 1000 kPa, and heating to 90 °C.

Subsequently, the reaction was started by adding the First Component of the catalytic composition. Pressure was maintained at 1000 kPa during the reaction by continuously recharging of consumed ethene.

At the end of the (predetermined) reaction time, the reaction was terminated by water injection. The product distribution was determined by gas-liquid chromatography.

The amount of ethene consumed during the reaction, the amounts of C₄, C₆-C₁₀ and C₁₂₊ olefins produced, and the wt% distribution of alpha-, beta- and branched hexenes are given in Table 1.

**TABLE 1**

| Example | 1 | 2 | 3 | 4 |
|---|---|---|---|---|
| Reaction time (min) | 3 | 103 | 102 | 23 |
| Ethene consumed (g) | 25 | 25 | 25 | 25 |
| Product C₄ olefin (g) | 0.9 | 2.0 | 1.6 | 1.2 |
| Product C₆₋₁₀ | 3.8 | 7.0 | 5.2 | 4.9 |
| olefins (g) | 3.8 | 7.0 | 5.2 | 4.9 |
| hexene (g) | 1.1 | 2.3 | 1.7 | 1.4 |
| Product C₁₂₊ olefins (g) | 20.3 | 16.0 | 18.2 | 18.9 |
| Distribution of hexenes (wt%) | | | | |
| 1-hexene | 81.8 | 57.9 | 43.5 | 98.8 |
| 2-hexene | 17.6 | 39.4 | 53.2 | 1.1 |
| 2-ethyl-1-butene | 0.7 | 2.7 | 3.3 | 0.1 |

### Examples 5 to 8: co-oligomerisation of ethene with pentene

The procedure in comparative Examples 5-7 and in Example 8 was the same as that of comperative Examples 1 to 3 and Example 4, respectively, except that the First and Second Components of the catalyst composition were dissolved in 15 and 60 ml of toluene respectively and that, together with the Second Component, 210 mmol of 1-pentene (co-monomer for reacting with the ethene) was injected into the autoclave. Comparative Example 7 differed in that the amounts of toluene and 1-pentene used were 300 ml and 91 mmol, respectively.

The amount of ethene consumed during the reaction, the amounts of C₄, C₆₊₈₊₁₀, C₇₊₉₊₁₁ and C₁₂₊ olefins produced, and the wt% distribution of alpha-, beta- and branched hexenes and heptenes are given in Table 2.

**TABLE 2**

| Exemple | 5 | 6 | 7 | 8 |
|---|---|---|---|---|
| Reaction time (min) | 4 | 150 | 30 | 38 |
| Ethene consumed (g) | 25 | 25 | 34 | 25 |
| Product C₄ olefin (g) | 0.2 | 0.8 | 5.0 | 1.2 |
| Product C₆₊₈₊₁₀ olefins (g) | 0.8 | 2.9 | 9.8 | 5.2 |
| hexene (g) | 0.2 | 1.0 | 4.3 | 1.6 |
| Product C₇₊₉₊₁₁ olefins (g) | 4.5 | 2.7 | 1.6 | 2.5 |
| heptene (g) | 1.4 | 1.0 | 0.8 | 0.8 |
| Product C₁₂₊ olefins (g) | 24.5 | 21.6 | 18.0 | 18.3 |
| Distribution of hexenes (wt%) | | | | |
| 1-hexene | 89.8 | 60.3 | 36.0 | 98.8 |
| 2 -hexene | 10.2 | 38.9 | 62.4 | 1.1 |
| 2-ethyl-1-butene | 0.0 | 0.8 | 1.6 | 0.1 |
| Distribution of heptenes (wt%) | | | | |
| 1-heptene | 86.3 | 57.6 | 36.2 | 97.6 |
| 2-heptene | 13.4 | 31.6 | 55.7 | 1.1 |
| 2-ethyl-1-pentene | 0.3 | 10.8 | 8.1 | 1.3 |

From these Examples it is apparent, that catalyst compositions according to the invention, when used in the oligomerisation of ethene (Example 4) and in the co-oligomerisation of ethene and pentene (Example 8), have the advantage above comparative catalyst compositions of promoting the selectivity of the reaction towards linear alpha-olefin reaction products and of suppressing the formation of branched and internal olefins.

## Claims

1. A catalyst composition comprising a first component which is a substituted-bis(cyclopentadienyl) titanium, zirconium or hafnium compound, also containing a substituent which is attached to the metal and which is capable of reacting with a cation and a second component which is a ionic combination of a bulky anion containing a plurality of boron atoms which is substantially non-coordinating under the reaction conditions employed, with a cation, characterized in that the substituted bis-cyclopentadienyl ligand pair is bis-tetramethylcyclopentadienyl.

2. A catalyst composition according to claim 1, characterized in that the first component of the catalyst is a compound of the general formula (Cp*)₂MR₁R₂ wherein (Cp*)₂ is the bis-tetramethylcyclopentadienyl ligand pair, M is a metal chosen from the group of titanium, zirconium or hafnium and R₁ and R₂ are two identical or different groups selected from unsubstituted or substituted hydrocarbyl groups, alkyloxy groups,hydrogen or halogen.

3. A catalyst composition according to claim 2, characterized in that R₁ and R₂ are unsubstituted alkyl groups of from 1 to 5 carbon atoms.

4. A catalyst composition according to claim 3, characterized in that the first component of the catalyst is bis(tetramethylcyclopentadienyl)zirconium dimethyl.

5. A catalyst composition according to claim 1, characterized in that the boron-containing non-coordinating anion is a carborate anion.

6. A catalyst composition according to claim 5, characterized in that the carborate anion is of the formula B₁₁CH₁₂-.

7. A catalyst composition according to claim 1, characterized in that the cation is a proton-donating cation.

8. A catalyst composition according to claim 7, characterized in that the proton-donating cation is a quaternary ammonium cation.

9. A catalyst according to claim 1, characterized in that the cation is a silver ion.

10. Use of a catalyst composition as claimed in any one of claims 1-9 for the preparation of linear alpha alkenes having 4-24 carbon atoms by oligomerisation or co-oligomerisation of ethene and/or an alpha alkene having 3-10 carbon atoms

## Patentansprüche

1. Katalysatorzusammensetzung, enthaltend eine erste Komponente, bei der es sich um eine Bis (cyclopentadienyl)titan-, -zirkonium- oder -hafniumverbindung mit substituierten Cyclopentadienylresten, die daneben auch einen an das Metall gebundenen Substituenten, der mit einem Kation reagieren kann, enthält, handelt, und eine zweite Komponente, bei der es sich um eine ionische Kombination aus einem sperrigen Anion mit mehreren Boratomen, das unter den Reaktionsbedingungen im wesentlichen nichtkoordinierend ist, und einem Kation handelt, dadurch gekennzeichnet, daß es sich bei dem substituierten Bis(cyclopentadienyl)-Ligandenpaar um Bis(tetramethylcyclopentadienyl) handelt.

2. Katalysatorzusammensetzung nach Anspruch 1, dadurch gekennzeichnet, daß es sich bei der ersten Komponente des Katalysators um eine Verbindung der allgemeinen Formel (Cp*)₂MR₁R₂ handelt, worin (Cp*)₂ das Bis(tetramethylcyclopentadienyl)-Ligandenpaar, M ein Metall, ausgewählt aus der Gruppe Titan, Zirkonium oder Hafnium, und R₁ und R₂ gleiche oder verschiedene Gruppen, ausgewählt aus gegebenenfalls substituierten Kohlenwasserstoffgruppen, Alkyloxygruppen, Wasserstoff oder Halogen, bedeuten.

3. Katalysatorzusammensetzung nach Anspruch 2, dadurch gekennzeichnet, daß R₁ und R₂ unsubstituierte Alkylgruppen mit 1 bis 5 Kohlenstoffatomen bedeuten.

4. Katalysatorzusammensetzung nach Anspruch 3, dadurch gekennzeichnet, daß es sich bei der ersten Komponente des Katalysators um Bis(tetramethylcyclopentadienyl)zirkoniumdimethyl handelt.

5. Katalysatorzusammensetzung nach Anspruch 1, dadurch gekennzeichnet, daß es sich bei dem borhaltigen, nichtkoordinierenden Anion um ein Carborat-Anion handelt.

6. Katalysatorzusammensetzung nach Anspruch 5, dadurch gekennzeichnet, daß das Carborat-Anion die Formel B₁₁CH₁₂ ⁻ hat.

7. Katalysatorzusammensetzung nach Anspruch 1, dadurch gekennzeichnet, daß es sich bei dem Kation um ein protonenabgebendes Kation handelt.

8. Katalysatorzusammensetzung nach Anspruch 7, dadurch gekennzeichnet, daß es sich bei dem protonenabgebenden Kation um ein quaternäres Ammoniumkation handelt.

9. Katalysator nach Anspruch 1, dadurch gekennzeichnet, daß es sich bei dem Kation um ein Silberion handelt.

10. Verwendung einer Katalysatorzusammensetzung gemäß einem der Ansprüche 1-9 bei einem Verfahren zur Herstellung von linearen alpha-Alkenen mit 4-24 Kohlenstoffatomen durch Oligomerisierung oder Cooligomerisierung von Ethen und/oder einem alpha-Alken mit 3-10 Kohlenstoffatomen.

## Revendications

1. Composition de catalyseur comprenant un premier composant qui est un composé de bis(cyclopentadiényl)titane, zirconium ou hafnium substitué, contenant également un substituant qui est fixé au métal et qui peut réagir avec un cation et un second composant qui est une combinaison ionique d'un anion volumineux contenant une pluralité d'atomes de bore qui est essentiellement non coordinant sous les conditions réactionnelles utilisées, avec un cation, caractérisée en ce que la paire de ligands de bis-cyclopentadiényle substitué est du bis-tétraméthylcydopentadiényle.

2. Composition de catalyseur suivant la revendication 1, caractérisée en ce que le premier composant du catalyseur est un composé de la formule générale
(Cp*)₂MR₁R₂,
dans laquelle (Cp*)₂ est la paire de ligands bis-tétraméthylcyclopentadiényle, M est un métal choisi dans le groupe comprenant le titane, le zirconium et l'hafnium et R₁ et R₂ sont deux groupes identiques ou différents choisis parmi les groupes hydrocarbyle non substitués et substitués, les groupes alcoxy, l'hydrogène et les atomes d'halogène.

3. Composition de catalyseur suivant la revendication 2, caractérisée en ce que R₁ et R₂ sont des groupes alkyle non substitués de 1 à 5 atomes de carbone.

4. Composition de catalyseur suivant la revendication 3, caractérisée en ce que le premier composant du catalyseur est du bis(tétraméthylcyclopentadiényl) zirconium diméthyle.

5. Composition de catalyseur suivant la revendication 1, caractérisée en ce que l'anion non coordinant contenant du bore est un anion carborate.

6. Composition de catalyseur suivant la revendication 5, caractérisée en ce que l'anion carborate est de la formule B₁₁CH₁₂-.

7. Composition de catalyseur suivant la revendication 1, caractérisée en ce que le cation est un cation donneur de protons.

8. Composition de catalyseur suivant la revendication 7, caractérisée en ce que le cation donneur de protons est un cation d'ammonium quaternaire.

9. Catalyseur suivant la revendication 1, caractérisé en ce que le cation est un ion d'argent.

10. Utilisation d'une composition de catalyseur suivant l'une quelconque des revendications 1-9 pour la préparation d'alpha-alcènes linéaires comportant 4-24 atomes de carbone par oligomérisation ou co-oligomérisation d'éthène et/ou d'un alpha-alcène comportant 3-10 atomes de carbone.
